# EUROPEAN PATENT SPECIFICATION

(11) **EP 3 480 275 B1**
(45) Date of publication and mention of the grant of the patent: **17.06.2020**
(21) Application number: 17761911.1
(22) Date of filing: 29.06.2017
(51) Int. Cl.: C09K 11/02, C09K 11/85

(54) **LUMINESCENTUPCONVERSION**
LUMINESZENTE AUFWÄRTSUMWANDLUNG
MATÉRIAUX LUMINESCENTS À CONVERSION ASCENDANTE ET PROCÉDÉ DE PRÉPARATION DE CES MATÉRIAUX

(30) Priority: 30.06.2016 ES 201630886
(43) Date of publication of application: 08.05.2019
(73) Proprietor: Innceinnmat, S.L., 12100 Grao de Castellon (Cast. de la Plana) (ES); Universitat de València, 46010 Valencia (ES); Consejo Superior de Investigaciones Cientificas, 28006 Madrid (ES)
(72) Inventor: PÉREZ PRIETO, Julia, E-46010 Valencia (ES); GONZÁLEZ BÉJAR, María, E-46010 Valencia (ES); ESTÉBANEZ BLOEM, Néstor Luis, E-46010 Valencia (ES); FERNÁNDEZ LOZANO, José Francisco, E-28049 Madrid (ES); ENRÍQUEZ PÉREZ, Esther, E-28049 Madrid (ES); LÓPEZ BUENDÍA, Ángel Miguel, E-46980 Paterna Valencia (ES); URQUIOLA CASAS, María del Mar, E-46100 Burjassot Valencia (ES)
(74) Representative: ABG Intellectual Property Law, S.L.
(86) International application number: PCT/ES2017/070474
(87) International publication number: WO 2018/002405

(56) References cited:
- EP-A1- 1 431 352
- WO-A1-2008/048190
- WO-A1-2009/054946
- WO-A1-2014/116631
- WO-A1-2015/102535
- US-A1- 2014 261 031
- US-A1- 2016 122 635

## Description

### Field of the Invention

The present invention relates to the area of luminescent upconversion materials. More specifically, the present invention relates to luminescent upconversion composite materials and nanoparticles and methods for preparing same.

### Background of the Invention

Upconversion processes consist of high-energy photon emission in the ultraviolet-visible-near-infrared range after sequential absorptions of lower energy photons, usually in the near-infrared range. The development of nanoparticles made up of the combination of a suitable crystalline matrix and certain trivalent lanthanide ions (Ln³⁺), which are capable of producing efficient upconversion emission (lanthanide-upconversion nanoparticles, Ln-UCNPs), such as for example, NaYF₄ doped with rare earth elements such as Er³⁺, Yb³⁺ and/or Tm³⁺, has become highly relevant today in the field of nanophotonics. This is due to the long photoluminescence half-lives of Ln³⁺ (from µs to several ms), and the low optical power density required for Ln-UCNP excitation, making it possible to use continuous diode lasers as an excitation source (much more cost-effective than the pulsed lasers normally required). Furthermore, Ln-UCNPs can emit at different wavelengths by selecting or combining Ln³⁺ ions, are rarely toxic and have an extraordinary photostability. These NaYF₄:Yb,Er(Tm)-type formulation materials have been known for their upconversion effect since the 1970s (Menyuk N. et al.; Appl. Phys. Lett., 1972, 21, 159; Sommerdijk J.L. and Bril A., Philips Tech. Rev. 1974, 34, 1) and were subsequently obtained in nanometric size (Zeng J. H. et al.; Adv. Mater. 2005, 17, 2119) .

All these advantages of Ln-UCNPs allow for a wide range of photonic applications as luminescent markers acting as security elements (WO 2014/090867 A2, Chen G. et al.; Chem. Soc. Rev., 2015, 44, 1680-1713) or imaging techniques in diagnosis and biomedicine (US 2009/0081461 A1), in which the use of near-infrared excitation allows for greater penetration depth of biological tissues, with minimum damage in living organisms. They are also applied in display and lighting device manufacture, and in white light generation technology, for which there is a need to incorporate Ln-UCNPs into hybrid composite materials by means of the dispersion or infiltration thereof in glass or polymeric materials, and by means of the deposition thereof as thin films on crystalline substrates, which materials are all transparent in the near-infrared range. White light generation by Ln-UCNP upconversion emission balance is an alternative to other white light systems such as LEDs, for example. EP 1 431 352 discloses nanoparticles dispersible in fluorine containing media.

The research and development of earlier photonic applications were made possible as a result of the development of a large variety of chemical pathways for preparing Ln-UCNPs including, mainly, co-precipitation (Wang L. Y. et al.; Angew. Chem., Int. Ed. 2005, 44(37), 6054-6057), thermal decomposition (Q. Li and Y. Zhang, Nanotechnology 2008, 19, 345606), solvothermal synthesis (Rhee H. W. et al.; J. Am. Chem. Soc. 2008, 130(3), 784-785), combustion synthesis (Liu Y. S. et al.; Chem. Soc. Rev. 2013, 42(16), 6924-6958) and sol-gel synthesis (Patra A. et al.; J. Phys. Chem. B 2002, 106(8), 1909-1912).

The Ln-UCNPs obtained in all these synthetic pathways are passivated with organic ligands anchored to the surface of the nanoparticles by means of non-covalent interactions and using anionic groups or with pairs of free electrons, such as carboxylate, phosphate, thiolate or amine groups. International patent application WO 2012/091778 A2 describes Ln-UCNPs of NaYF₄:Yb,Er, Gd₂O₂S:Yb,Er, Y₂O₃:Yb,Er, CeO₂ or NaGdF₄:Tb, all of them oleate-passivated. US patent application 2010/0261263 A1 describes Ln-UCNPs which have a core/shell structure comprising a Y₂O₃:Ln core with a NaYF₄ shell and passivated with polyethylene glycol or polyethyleneimine. Ligands are essential for controlling the size and shape of Ln-UCNPs during the synthesis process thereof in dissolution, as well as for preventing the nanoparticles from collapsing, and therefore from precipitating.

The Ln-UCNPs obtained by means of these methods are generally hydrophobic, this being a drawback for many applications for which the Ln-UCNPs must be able to be dispersed in aqueous media, for example, for the application thereof in biological samples. This drawback is usually solved in the state of the art by means of ligand exchange or performing passivation with macromolecules such as polymers (Muhr V. et al.; Acc. Chem. Res., 2014, 47 (12), 3481-3493) or oxidizing the ligand (Naccache R. et al.; Chem. Mater., 2009, 21(4), 717-723) in order to make the Ln-UCNPs hydrophilic.

However, organic ligand passivation of the Ln-UCNPs described in the state of the art has a low stability in acidic media because the ligands used separate from the surface of the nanoparticle at acidic pHs (Liras M. et al.; Chem. Mater. 2014, 26, 4014-4022). In fact, carboxylate ligands anchored to Ln-UCNPs are usually removed by means of protonating same at pH 4 approximately (Bogdan N. et al.; Nano Lett., 2011, 11, 835-840). This low organic passivation stability at acidic pHs entails a drawback for the use of nanoparticles of this type in many applications that require acidic media, such as for example, the synthesis of ceramic composite materials by means of the sol-gel technique such as those required in the present invention.

Therefore, despite the large variety of Ln-UCNPs and Ln-UCNP synthesis pathways described in the state of the art, there is still a need to obtain new Ln-UCNPs that are stable in acidic media and have a high upconversion efficiency. These new Ln-UCNPs must also be applicable as particulate or film materials, maintaining their transparency and preferably with ceramic-type stability and hardness. Although the preparation of ceramic coatings by means of the sol-gel technique has been described such as in WO 2012/113953 A1, for example, there is still a need in the state of the art for ceramic coatings comprising Ln-UCNPs with high luminescent efficiency.

### Brief Description of the Invention

The authors of the present invention have developed nanoparticles which have upconversion luminescent properties and are stable in extremely acidic media as a result of the organic ligands binding strongly to the surface of the nanoparticles. Furthermore, the nanoparticles of the invention are soluble in aqueous medium and can be conjugated to other molecules, and therefore change the interaction thereof with the medium (solubility) or incorporate functional molecules.

Therefore, a first aspect of the invention relates to nanoparticles of formula MLnF₄:RE⁺³, where M is an alkaline metal, Ln is a lanthanide element or an analog thereof and RE⁺³ is an ion of rare earth elements or combinations thereof, characterized in that said nanoparticles comprise organic ligands bound to the surface thereof by means of covalent bonds.

The nanoparticles of the invention have upconversion luminescent properties and are synthesized from naked precursor nanoparticles (UCNP_{naked}), characterized in that they comprise on their surface -O⁻, -OH or -HOH groups that are derived from water molecules present in the synthesis medium. First, UCNP_{naked} are obtained by means of treatment, in acidic medium, of UCNPs passivated with ligands such as oleate or other carboxylates, for example. The functionality of the UCNP_{naked} surface (-O⁻, -OH or -HOH group) is therefore utilized for obtaining the nanoparticles of the invention (UCNP@ligand, wherein "ligand" refers to an organic compound bound by means of a stable covalent bond to the surface of the nanoparticles).

Therefore, another aspect of the present invention provides a method for preparing luminescent upconversion nanoparticles of formula MLnF₄:RE⁺³, where M is an alkaline metal, Ln is a lanthanide element or an analog thereof and RE⁺³ is an ion of rare earth elements or combinations thereof, characterized in that said nanoparticles comprise organic ligands bound to the surface thereof by means of covalent bonds, which method comprises the steps of:
a) adding to a polar solvent precursor nanoparticles of formula MLnF₄:RE⁺³, where M is an alkaline metal, Ln is a lanthanide element or an analog thereof and RE⁺³ is an ion of rare earth elements or combinations thereof, characterized in that said nanoparticles comprise on their surface -O⁻, -OH, -HOH groups or combinations thereof;
b) adding a base in excess to the mixture resulting from step (a); and
c) after removing the excess base from the mixture resulting from step (b), adding a compound of formula X-CH₂CO-Z, where X is a halogen, Z is -H, -R, -COR, -OH, -OCOR, -NH₂, -NHR, -NR₂,-NHCOR, -SR or -SCOR, and R is a linear or branched alkyl chain.

An additional aspect of the invention relates to nanoparticles of formula MLnF₄:RE⁺³, where M is an alkaline metal, Ln is a lanthanide element or an analog thereof and RE⁺³ is an ion of rare earth elements or combinations thereof, characterized in that said nanoparticles comprise organic ligands bound to the surface thereof by means of covalent bonds and can be obtained by means of the preparation method as defined above.

Another additional aspect of the present invention relates to the use of the luminescent upconversion nanoparticles as defined above as fluorescent markers or photosensitizers.

The present invention further provides a luminescent composite material comprising the luminescent upconversion nanoparticles as defined above. The combination of both components (matrix and nanoparticles) enhances the upconversion luminescent properties and the stability of the nanoparticles, and provides a composite material with excellent upconversion luminescent performance.

Therefore, another aspect of the present invention relates to a luminescent composite material comprising the luminescent upconversion nanoparticles as defined above.

An additional aspect of the present invention relates to the use of the luminescent composite material as defined above as a fluorescent marker or a photosensitizer.

Finally, another additional aspect of the present invention relates to the use of the luminescent composite material as defined above in the paper, plastic, packaging, textile, automotive, paint, biological, medical-pharmaceutical, document security, tracer, ceramic pigment, optoelectronics or photovoltaic sectors.

### Drawings

Figure 1 shows the comparison of the FTIR spectra for UCNP@ligand_{2-chloroacetamide} at different pHs, where UCNP is NaYF₄: Yb, Er.
Figure 2 shows the comparison of the emission spectra for UCNP@ligand_{2-chloroacetamide} at different pHs, where UCNP is NaYF₄: Yb, Er and where the pH of a) is 7.53 (control), the pH of b) is 2.00, the pH of c) is 2.00 after 2 hours, and the pH of d) is 7.53 after 48 hours.
Figure 3 shows the C₁ₛ XPS spectrum for 2-chloroacetamide (top) and for UCNP@ligand_{2-chloroacetamide} (bottom), where UCNP is NaYF₄: Yb, Er.
Figure 4 shows the N₁ₛ XPS spectrum for 2-chloroacetamide (left) and for UCNP@ligand_{2-chloroacetamide} (bottom), where UCNP is NaYF₄: Yb, Er.
Figure 5 shows the C₁ₛ XPS spectrum for 2-chloroacetic acid (top) and for UCNP@ligand_{2-chloroacetic acid} (bottom), where UCNP is NaYF₄: Yb, Er.
Figure 6 shows the O₁ₛ XPS spectrum for 2-chloroacetic acid (top) and for UCNP@ligand_{2-chloroacetic acid} (bottom), where UCNP is NaYF₄: Yb, Er.
Figure 7 shows the luminescence of the composite material comprising UCNP@ligand_{2-chloroacetamide}, where UCNP is NaYF₄: Yb, Er, encapsulated by means of a sol-gel method.

### Detailed Description of the Invention

The main object of the present invention is to provide luminescent upconversion nanoparticles, more specifically nanoparticles of formula MLnF₄:RE⁺³, where M is an alkaline metal, Ln is a lanthanide element or an analog thereof and RE⁺³ is an ion of rare earth elements or combinations thereof, characterized in that said nanoparticles comprise organic ligands according to claim 1 bound to the surface thereof by means of covalent bonds, as well as composite ceramic materials with synergistic properties containing said nanoparticles.

Upconversion processes consist of high-energy photon emission in the ultraviolet-visible-near-infrared range after sequential absorptions of lower energy photons.

Therefore, the nanoparticles of the invention have upconversion luminescent properties with emission in the visible-ultraviolet-near-infrared range after multiple excitations with lower energy photons, particularly in the near-infrared range.

The nanoparticles of the invention can have a cubic, hexagonal, spherical, tetragonal, rhomboid, monoclinic, triclinic structure or a combination thereof. The nanoparticles of the invention preferably have a mean size less than or equal to 500 nm, even more preferably between about 20 nm and about 500 nm.

The nanoparticles of the invention have a matrix of formula MLnF₄ doped with trivalent ions of rare earth elements RE³⁺ or combinations thereof, and where M is an alkaline metal and Ln is a lanthanide element or an analog thereof.

In a preferred embodiment, M is Na or Li. In another preferred embodiment, RE³⁺ is Yb³⁺, Er³⁺, Tm³⁺, Gd³⁺ or mixtures or combinations thereof.

The term "lanthanide element" refers to a period 6 element of the periodic table selected from La, Ce, Pr, Nd, Pm, Sm, Eu, Gd, Tb, Dy, Ho, Er, Tm, Yb and Lu, preferably Yb.

The term "analog thereof" refers to an element located within group 3 of the periodic table and is chemically similar to a lanthanide element. In a preferred embodiment, the analog element is Y.

A non-limiting example of matrix is NaYF₄ doped with Yb³⁺, Er³⁺, Tm³⁺, Gd³⁺ or combinations thereof.

In a particular embodiment, the nanoparticles of the invention have a core/shell structure. The term "core/shell structure" refers to nanoparticles comprising a core of an inner material and a shell of an outer material. The nanoparticles which can be obtained by means of the preparation method as defined above with a core/shell structure can have the same or a different chemical composition for the inner material of the core and for the outer material of the shell. A non-limiting example of nanoparticles of the invention with a core/shell structure is NaYF₄:RE³⁺/NaYF₄:RE³⁺ or NaYF₄:RE³⁺/NaYF₄.

The nanoparticles of the invention further comprise organic ligands bound by covalent bonds to the surface thereof, which ligands comprise a -CH₂CO- unit. This short unit allows the ligands to be strongly bound to the surface of the nanoparticles of the invention even in extremely acidic media, unlike groups linked by means of coordination or ionic bonds described in the state of the art that separate from of the surface of the nanoparticles in media with acidic pHs.

The term "covalent bond" refers to a bond that takes place between two atoms having a difference in electronegativity of less than 1.7 between them, and in which said atoms reach the stable octet, sharing last level electrons.

Therefore, in a particular embodiment the nanoparticles of the invention comprise organic ligands bound to the surface thereof by means of covalent bonds that are resistant to highly acidic pHs, preferably to pHs between about 1.0 and about 4.0.

Another aspect of the present invention provides a method for preparing luminescent upconversion nanoparticles of formula MLnF₄:RE⁺³, where M is an alkaline metal, Ln is a lanthanide element or an analog thereof and RE⁺³ is an ion of rare earth elements or combinations thereof, characterized in that said nanoparticles comprise organic ligands bound to the surface thereof by means of covalent bonds, which method comprises the steps of:
a) adding to a polar solvent precursor nanoparticles of formula MLnF₄:RE⁺³, where M is an alkaline metal, Ln is a lanthanide element or an analog thereof and RE⁺³ is an ion of rare earth elements or combinations thereof, characterized in that said nanoparticles comprise on their surface -O⁻, -OH, -HOH groups or combinations thereof;
b) adding a base in excess to the mixture resulting from step (a); and
c) after removing the excess base from the mixture resulting from step (b), adding a compound of formula X-CH₂CO-Z, where X is a halogen, Z is -H, -R, -COR, -OH, -OCOR, -NH₂, -NHR, -NR₂,-NHCOR, -SR or -SCOR, and R is a linear or branched alkyl chain.

Therefore, the method for preparing nanoparticles of the invention comprises step (a) which involves adding to a polar solvent precursor nanoparticles of formula MLnF₄:RE⁺³, where M is an alkaline metal, Ln is a lanthanide element or an analog thereof and RE⁺³ is an ion of rare earth elements or combinations thereof, characterized in that said nanoparticles comprise on their surface -O⁻, -OH, -HOH groups or combinations thereof.

In a preferred embodiment, M is Na or Li. In another preferred embodiment, RE³⁺ is Yb³⁺, Er³⁺, Tm³⁺, Gd³⁺ or mixtures or combinations thereof. In a preferred embodiment, Ln is Y.

A non-limiting example of the matrix of the nanoparticles of the invention is NaYF₄ doped with Yb³⁺, Er³⁺, Tm³⁺, Gd³⁺ or combinations thereof.

Non-limiting examples of polar solvents suitable for step (a) of the method of the invention are dimethylformamide (DMF), acetonitrile (ACN), tetrahydrofuran (THF) or a mixture thereof.

In another preferred embodiment, step (a) of the method of the invention takes place at a temperature from about 25°C to about 80°C.

The method for preparing nanoparticles of the invention further comprises step (b) which comprises adding a base in excess to the mixture resulting from step (a) as defined above.

Bases suitable for step (b) of the method of the invention are inorganic bases, such as for example, MOH-type bases where M is an alkaline metal, such as NaOH, KOH, LiOH or a mixture thereof.

In a preferred embodiment, the base in excess of step (b) of the method of the invention is an inorganic base, even more preferably NaOH.

In another preferred embodiment, the base of step (b) of the method of the invention is added in excess between 10% and 50% by weight.

The method of the invention further comprises step (c) which consists of adding, after removing the excess base from the mixture resulting from step (b) as defined above, an organic compound of formula X-CH₂CO-Z, where X is a halogen, Z is -H,-R, -COR, -OH, -OR, -OCOR, -NH₂, -NHR, -NR₂, -NHCOR, -SR or -SCOR, and where R is a linear or branched alkyl chain.

The compound of formula X-CH₂CO-Z, which comprises a halogen that is directly bound to the alpha carbon with respect to the carbonyl group (CO) and is located a distance of exactly two carbons (CH₂CO) from Z, reacts with the reactive groups on the surface of the nanoparticles (-O⁻, -OH or -HOH), forming a strong chemical bond (covalent bond) between them and allowing the ligand to be strongly bound to the surface of the nanoparticles of the invention even in extremely acidic media.

Group Z of the compound of formula X-CH₂CO-Z provides functionality to the surface of the nanoparticles of the invention, which allows the conjugation thereof with other functional molecules and/or the interaction thereof with the medium (solubility). In a preferred embodiment, Z is -H, -NH₂ or -OCOR.

Group R is a linear or branched alkyl chain. Non-limiting examples of R groups suitable for step (c) of the method of the invention are -CH₃, -CH₂CH₃, -CH₂CH₂CH(CH₃)₂.

According to the invention compounds of formula X-CH₂CO-Z suitable for step (c) of the method of the invention are 2-chloroacetamide, 2-bromoacetamide, 2-chloroacetic acid, 2-bromoacetic acid, and ethyl 2-chloroacetate, preferably 2-chloroacetamide and 2-chloroacetic acid.

In a particular embodiment, the method for preparing nanoparticles as defined above comprises an additional step (d) which comprises precipitating the nanoparticles obtained in step (c). The precipitation of the nanoparticles of step (d) of the method of the invention is preferably performed by means of centrifugation.

In yet another more particular embodiment, step (d) of the method of the invention further comprises washing and drying the precipitate.

Therefore, the nanoparticles obtained through the method of the present invention are stable in extremely acidic media because the reaction of the compound of formula X-CH₂CO-Z with the reactive groups of the surface of the nanoparticles gives rise to a covalent bond between them and allows the ligand to remain strongly bound to the surface of the nanoparticles even in extremely acidic media.

Therefore, an additional aspect of the present invention relates to luminescent upconversion nanoparticles of formula MLnF₄:RE⁺³, where M is an alkaline metal, Ln is a lanthanide element or an analog thereof and RE⁺³ is an ion of rare earth elements or combinations thereof, characterized in that said nanoparticles comprise organic ligands bound to the surface thereof by means of covalent bonds and can be obtained by means of the preparation method as defined above.

The nanoparticles which can be obtained by means of the preparation method as defined above have upconversion luminescent properties with emission in the visible-ultraviolet-near-infrared range after multiple excitations with lower energy photons, particularly in the near-infrared range.

The nanoparticles which can be obtained by means of the preparation method as defined above can have a cubic, hexagonal, spherical, tetragonal, rhomboid, monoclinic, triclinic structure or a combination thereof. The nanoparticles which can be obtained by means of the preparation method as defined above preferably have a mean size less than or equal to 500 nm, even more preferably between about 20 nm and about 500 nm.

The nanoparticles which can be obtained by means of the preparation method as defined above have a matrix of formula MLnF₄ doped with trivalent ions of rare earth elements RE³⁺ or combinations thereof and where M is an alkaline metal and Ln is a lanthanide element or an analog thereof.

In a preferred embodiment, M is Na or Li. In another preferred embodiment, RE³⁺ is Yb³⁺, Er³⁺, Tm³⁺, Gd³⁺ or combinations thereof. In a preferred embodiment, Ln is Y.

A non-limiting example of the matrix of the nanoparticles of the invention is NaYF₄ doped with Yb³⁺, Er³⁺, Tm³⁺, Gd³⁺ or combinations thereof.

In a particular embodiment, the nanoparticles which can be obtained by means of the preparation method as defined above have a core/shell structure. The term "core/shell structure" refers to nanoparticles comprising a core of an inner material and a shell of an outer material. The nanoparticles which can be obtained by means of the preparation method as defined above with a core/shell structure can have the same or a different chemical composition for the inner material of the core and for the outer material of the shell. A non-limiting example of nanoparticles which can be obtained by means of the preparation method as defined above with a core/shell structure is NaYF₄:RE³⁺/NaYF₄ or NaYF₄:RE³⁺/NaYF₄:RE³⁺.

The nanoparticles which can be obtained by means of the preparation method as defined above are characterized in that they further comprise organic ligands covalently bound to their surface, which ligands comprise a -CH₂CO- unit. This short unit allows the ligands to be strongly bound to the surface of the nanoparticles which can be obtained by means of the preparation method as defined above, even in extremely acidic media.

Therefore, in a particular embodiment the nanoparticles which can be obtained by means of the preparation method as defined above are characterized in that they comprise organic ligands bound to the surface thereof by means of covalent bonds that are resistant to strongly acidic pHs, preferably to pHs between about 1.0 and about 4.0.

Furthermore, the nanoparticles of the present invention are stable when dispersed in aqueous or organic media, integrated in a masterbatch material or in the form of fine powder, providing versatility for dosing and storing same.

Additionally, the nanoparticles of the present invention have a low toxicity and can be conjugated with bio-compatible molecules, making them compatible for applications as a fluorescent marker or photosensitizers for tumors, for example, in the biological or medical-pharmaceutical sector.

Therefore, another aspect of the present invention relates to the use of the luminescent upconversion nanoparticles as defined above as fluorescent markers or photosensitizers, preferably in the biological or medical-pharmaceutical sector.

The present invention further provides a luminescent composite material comprising the luminescent upconversion nanoparticles as defined above.

In a preferred embodiment, the luminescent composite material as defined above is transparent.

The term "transparent" refers to a material that readily allows the passage of light in the visible range.

The term "composite material" refers to combinations of at least two types of materials for achieving the combination of properties that cannot be obtained in original materials. These composite materials have a continuous matrix responsible for the physical and chemical properties, and a discrete load.

In the composite material of the present invention, and as mentioned above, the combination of both materials (matrix and nanoparticles) gives rise to a synergy effect since it enhances the upconversion luminescent properties and the stability of the nanoparticles, and provides a composite material with excellent upconversion luminescent performance.

In a particular embodiment, the luminescent upconversion nanoparticles comprised in the composite material of the present invention have an emission capacity of more than 100-fold with respect to the original luminescent upconversion nanoparticles, and of more than 600-fold in an even more preferred embodiment.

In another particular embodiment, the luminescent composite material of the present invention is a ceramic composite material. Said material comprises the nanoparticles as defined above encapsulated in a ceramic matrix.

In a preferred embodiment, the luminescent composite material of the present invention is a ceramic composite material which can be obtained by means of a sol-gel synthesis process. A "sol-gel synthesis process" is understood to be those chemical pathways that are widely known in the field of the art of the present invention and comprise the synthesis of a colloidal suspension of solid particles in a liquid (referred to as sol) and the hydrolysis and condensation of said sol to form a solvent-filled solid material (gel).

In another preferred embodiment, the sol-gel synthesis process comprises the following steps:
i) dispersing the luminescent upconversion nanoparticles as defined above in a polar solvent comprising a metal alkoxide precursor, a dispersing agent and a surface active agent;
ii) adding water to the dispersion of step (i) to cause a hydrolysis and sol formation reaction;
iii) allowing the sol of step (ii) to condense, causing gel formation;
iv) aging and drying the gel obtained in step (iii) to yield a luminescent ceramic composite material; and
v) making the composite material resulting from step (iv) denser by means of heat treatment.

Step (i) of the sol-gel synthesis process of the present invention comprises dispersing the luminescent upconversion nanoparticles of the invention in a polar solvent comprising a metal alkoxide precursor, a dispersing agent and a surface active agent, and optionally, a drying retarder.

In the context of the present invention, the term "dispersing agent" refers to an additive that keeps the dispersion of step (i) of the sol-gel synthesis process of the invention stable. Non-limiting examples of dispersing agents suitable for the sol-gel synthesis process of the present invention are polyacrylates such as polyacrylic acid.

In the context of the present invention, the term "surface active agent" refers to an additive which modifies the contact surface between two phases and favors wetting the nanoparticles. The surface active agent also includes a film-forming or leveling agent such as polyester-modified polydimethylsiloxane.

The dispersion of step (i) is obtained using high-speed shearing processes. Non-limiting examples of the high-speed shearing dispersion suitable for step (i) of the sol-gel synthesis process of the present invention are Cowless-type dispersion, dispersion using rotor-stator systems or systems such as attrition milling with microbeads. The media used must be effective to favor the dispersion of the nanoparticles in the medium, and if agglomerates are present, they must have a size less than 200 nm.

In a preferred embodiment, the polar solvent is an alcohol or mixture of alcohols, preferably a primary alcohol or mixture of primary alcohols.

In another preferred embodiment, the luminescent upconversion nanoparticles of step (i) are dispersed at a concentration between 0.1% and 10% by weight, preferably about 4% by weight.

In the context of the present invention, "metal alkoxide" is understood to be a chemical compound comprising a metal atom M bound at least to an organic group through an oxygen atom (M-OR). The metal alkoxide is formed *in situ* or *ex situ* from an inorganic-type precursor such as metal nitrates, chlorides or perchlorates, or from an organic-type precursor such as acetates or acetylacetonates. Non-limiting examples of metal alkoxides suitable for the sol-gel synthesis process of the present invention when the metal is, for example, silicon are tetraethyl orthosilicate (TEOS), methyltriethoxysilane (MTES), methyltrimethoxysilane, 3-glycidoxypropyltrimethoxysilane or vinyltriethoxysilane.

Depending on the characteristics desired for the final composite material, the sol-gel synthesis process is performed via the acidic route, preferably using a silicon alkoxide precursor, such as silicon chloride, for example, or via a basic route, preferably using a zinc alkoxide precursor, such as zinc acetate, for example. The nature of the alkoxide will determine the pH range in which sol condensation and gel formation will take place (step (iii)).

The dispersion of step (i) of the sol-gel synthesis process of the present invention can optionally comprise a drying retarder.

Unless otherwise indicated, the listed components of the dispersion of step (i) are well-known known by the person skilled in the art for sol-gel synthesis processes and can be of an organic or inorganic type.

The sol-gel synthesis process of the present invention further comprises a step (ii) of adding water to the dispersion of step (i) to cause a hydrolysis and sol formation reaction.

In a particular embodiment, step (ii) further comprises adding a catalyst which speeds up the hydrolysis reaction. Non-limiting examples of catalysts suitable for the sol-gel synthesis process of the present invention are of the inorganic type, such as hydrochloric acid, for example, and of the organic type, such as ethanolamine, for example.

The sol-gel synthesis process of the present invention further comprises a step (iii) of allowing the sol of step (ii) to condense to cause gel formation. The gel that is obtained acts as an element for encapsulating the luminescent upconversion nanoparticles of the present invention, such that it prevents the aggregation of said nanoparticles.

In a particular embodiment, gel formation in step (iii) takes place at a pH between ≤6 and ≥2. The pH can be determined by means of the acidic or basic nature of the metal alkoxide used in step (i) as defined above.

The sol-gel synthesis process of the present invention further comprises a step (iv) of aging the gel obtained in step (iii).

Aging the gel favors gel homogeneity. A non-limiting example of the aging of a gel obtained in step (iii) is the use of zinc acetate as a metal alkoxide precursor by means of heating at 60°C for 72 hours in a closed flask to favor Zn(II) ion hydrolysis and condensation processes.

Step (iv) of the sol-gel synthesis process of the present invention further comprises drying the aged gel to remove the solvent, yielding a luminescent composite material, and comprises the heat treatment thereof in a range of temperatures comprised between 30°C and 200°C. This process is carried out, for example, on a crystallization tray in an oven at 60°C for 24 hours.

The sol-gel synthesis process of the present invention further comprises a step (v) of making the ceramic composite material resulting from step (iv) denser by means of heat treatment, removing the organic residues of the precursors. The heat treatment is carried out at a temperature between 300°C and 600°C with a residence time between 0.1 to 24 hours.

In a particular embodiment, the sol-gel synthesis process of the present invention further comprises a step (vi) of conditioning the resulting composite material by means of milling to obtain a particle size in a desired range of sizes. A non-limiting example consists of dry milling in a mixing mill using stabilized zirconium beads 1 mm in diameter for 30 minutes to obtain a powdery material consisting of the luminescent nanoparticles of the invention dispersed in a sol-gel matrix that has been made denser and has an average size where 90% of the particles have a size less than 10 µm.

In a particular embodiment, the luminescent composite material of the invention has a high resistance to chemical agents and a high thermal stability, preferably at temperatures ≥400°C. These properties allow the application thereof as security markers, forming part of paints, in the preparation of functional ceramic materials or forming part of coating for large surfaces. In another preferred embodiment, the luminescent composite material is colorless, which allows the application thereof as an authentication or quality marker or in sectors such as the paper, packaging or textile sector.

Therefore, a final aspect of the present invention relates to the use of the luminescent composite material as defined above in the paper, plastic, packaging, textile, automotive, biological, medical-pharmaceutical, document security, tracer, ceramic pigment, optoelectronics or photovoltaic sectors.

### Examples

The first part of the experimental section (Examples 1 to 3) refers to the preparation of the nanoparticles of the present invention. The second part of the experimental section (Examples 4 to 7) refers to the evaluation of the stability of the binding of the ligand to the surface of the nanoparticles of the present invention in acidic media, which is of interest for sol-gel encapsulation, among other applications. The third part of the experimental section (Examples 8 and 9) refers to the preparation of the composite material of the present invention.

### Example 1: Oleate-passivated NaYF₄:Yb, Er nanoparticle (UCNP@oleate) synthesis

NaYF₄:Yb³⁺, Er³⁺ nanoparticles were synthesized following the process described by Frances-Soriano et al. (Nanoscale 2015, 7, 5140 - 5146). Particularly, a mixture of 0.8 mmol of YCl₃·6H₂O, 0.18 mmol of YbCl₃·6H₂O, 0.02 mmol of ErCl₃·6H₂O (0.02 mmol), 12 mL of oleic acid and 15 mL of octadecene (ODE) was heated at 160°C in a 50 mL flask under continuous stirring. After the dissolution of the lanthanide salts (approx. 30 minutes), the mixture was cooled at 110°C and 10 mL of a solution consisting of methanol, NaOH (2.5 mmol) and NH₄F (4.0 mmol) were then slowly added. The mixture was degassed at 100°C for 30 minutes under continuous stirring. Finally, the mixture was heated at 305°C under N₂ atmosphere for 1 hour. The solution was then cooled to room temperature and the nanoparticles precipitated by means of centrifugation (10000 rpm, 10 minutes, 25°C). The oleate-passivated nanoparticles were washed three times with (43.5:40.5:16 v/v) hexane/acetone/methanol solutions.

### Example 2: Naked NaYF₄:Yb,Er nanoparticle (UCNP_{naked}) synthesis

Naked NaYF₄:Yb³⁺, Er³⁺ nanoparticles were prepared following the method by Capobianco et al. (Nano Lett. 2011, 11, 835-840). Particularly, 100 mg of UCNP@oleate were dispersed in 10 mL of aqueous HCl at pH 4. The mixture was stirred for 2 hours and the solution was kept at pH 4 by adding 0.1 M HCl. The oleic acid was removed by means of diethyl ether extraction (3 times). All the ether phases were pooled and a re-extraction was performed with water. The oleic acid was combined with all the aqueous phases and re-extracted with diethyl ether. UCNP_{naked} were recovered from the aqueous phase by precipitation with acetone followed by centrifugation (10000 rpm, 15 minutes, 25°C). The precipitate was washed three times with acetone and reprecipitated by centrifugation. Finally, the UCNP_{naked} were dispersed in Milli Q water.

### Example 3: 2-chloroacetamide-passivated NaYF₄:Yb,Er nanoparticle (UCNP@ligand_{2-chloroacetamide}) synthesis

The UCNP_{naked} nanoparticles were dispersed in DMF, the medium was made alkaline with sodium hydroxide, and after separating the excess NaOH, the solid ligand, 2-chloroacetamide, was added to the colloidal solution. After stirring for 48 hours, precipitation of the passivated nanoparticles (UCNP@ligand_{2-chloroacetamide}) was induced by centrifugation, the solid was washed several times and finally dried.

### Example 4: 2-chloroacetic acid-passivated NaYF₄:Yb,Er nanoparticle (UCNP@ligand_{2-chloroacetic}) synthesis

The UCNP_{naked} nanoparticles were dispersed in DMF, the medium was made alkaline with sodium hydroxide, and after separating the excess NaOH, the solid ligand, 2-chloroacetic acid, was added to the colloidal solution. After stirring for 48 hours, precipitation of the passivated nanoparticles (UCNP@ligand_{2-chloroacetic}) was induced by centrifugation, the solid was washed several times and finally dried.

### Example 5: UCNP@ligand_{2-chloroacetamide} stability in acidic medium

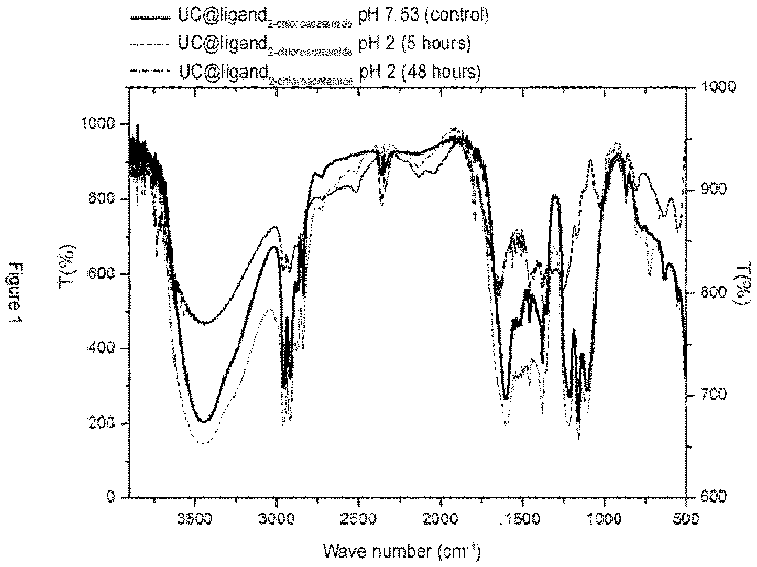

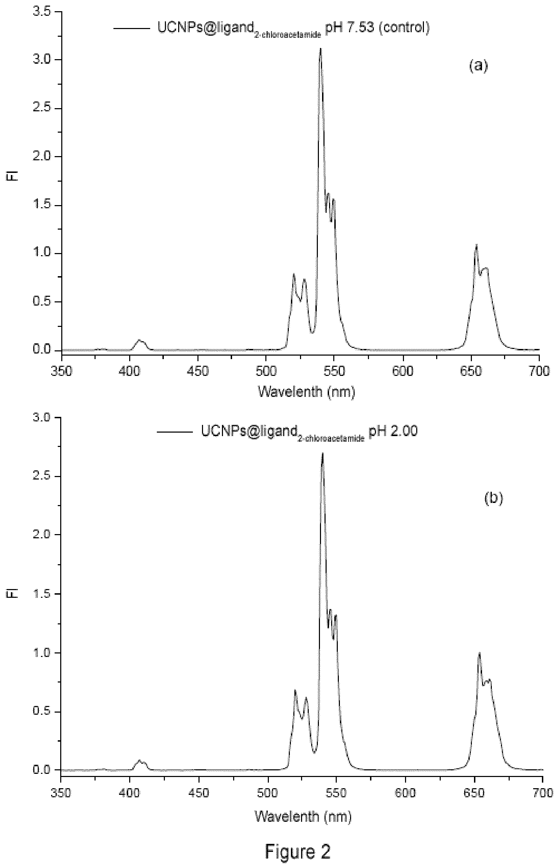

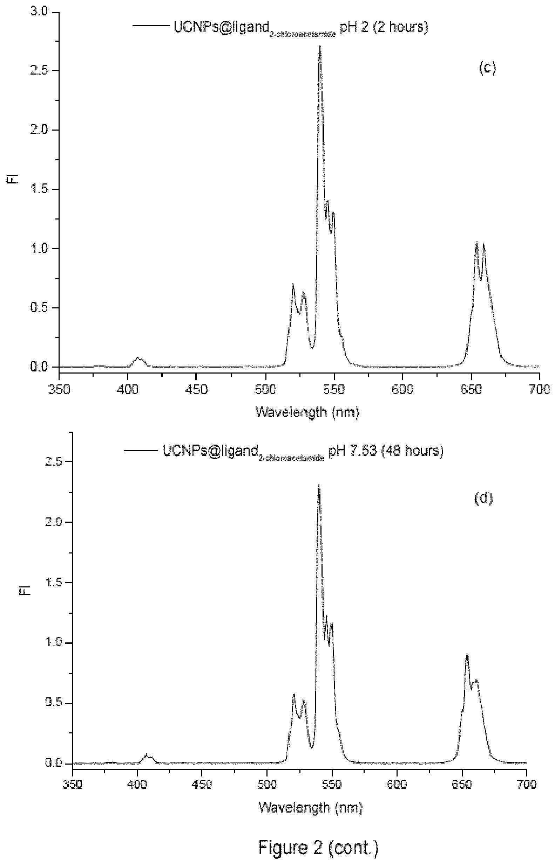

The FTIR (Fourier transform infrared spectroscopy) spectra were obtained for the UCNP@ligand_{2-chloroacetamide} nanoparticles (Figure 1) prepared as described in Example 3, and after 5 and 48 hours in aqueous dissolution at pH 2. The UCNP@ligand_{2-chloroacetamide} nanoparticles showed insignificant variation of their emission properties when they are dispersed in water at pH 2 for 48 hours, as can be confirmed in Figure 2b. The UCNP@ligand_{2-chloroacetamide} nanoparticles were then precipitated by means of causing them to have a neutral pH by adding NaOH and centrifuging same; they were washed with water and dried. The FTIR analysis of said nanoparticles (Figure 1) demonstrates that the organic ligand does not separate from the UCNP@ligand_{2-chloroacetamide} nanoparticles and its emission properties remain unchanged (Figures 2a-2d).

### Example 6: XPS characterization of UCNP@ligand_{2-chloroacetamide} nanoparticles

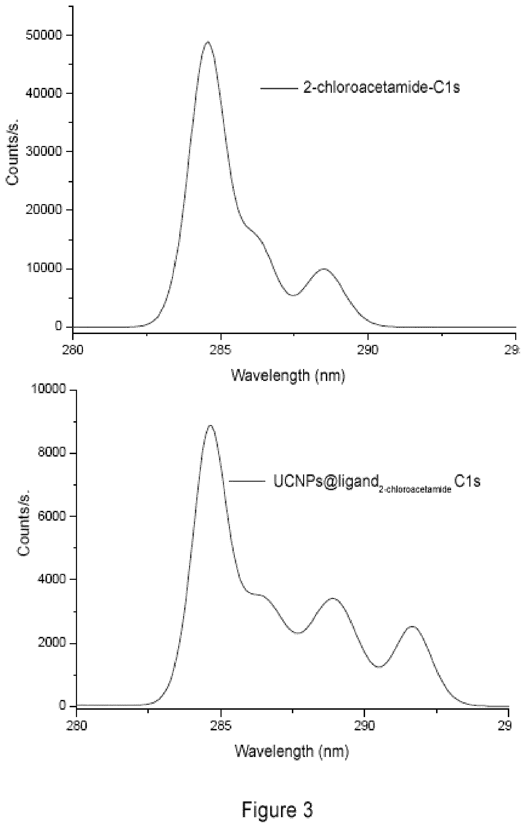

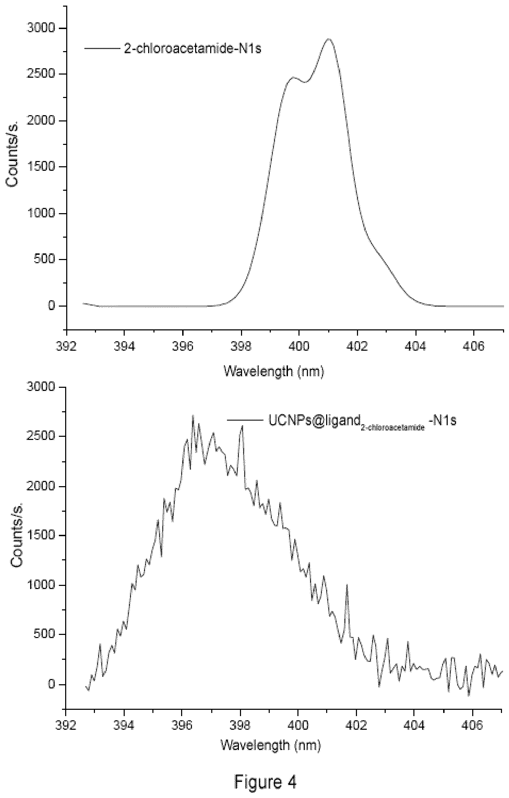

To compare of the free and bound ligand, the C₁ₛ XPS spectra for UCNP@ligand_{2-chloroacetamide} nanoparticles (Figure 3, bottom) prepared as described in Example 3 and for the free starting ligand, i.e., 2-chloroacetamide (Figure 3, top), were obtained. The N₁ₛ XPS spectra for UCNP@ligand_{2-chloroacetamide} nanoparticles (Figure 4, bottom) prepared as described in the Example 3 and for the organic starting compound, i.e., 2-chloroacetamide (Figure 4, top), were also obtained.

The significant changes in the C₁ₛ and N₁ₛ energy values between the organic starting compound, 2-chloroacetamide, and the organic ligand of UCNP@ligand_{2-chloroacetamide} must be pointed out. These shifts are consistent with a chemical reaction between the organic starting compound, 2-chloroacetamide, and the surface of the nanoparticle which allows the ligand to remain on the surface of the nanoparticle in strongly acidic media.

However, the C₁ₛ XPS spectra for UCNP@oleate nanoparticles known in the state of the art and prepared according to Example 1 show small changes in relation to the free oleic acid. The same is also observed for different carboxylic acids, such as, decanoic acid or dodecanedioic acid (Liu Y. et al. Nanoscale 2011, 3, 4804), for example, and demonstrates that the ligand is adsorbed on the surface of the nanoparticle through the carboxylate group. The small energy variation of the functional group which is adsorbed on the surface of the nanoparticle is a general issue for nanoparticles described in the state of the art as shown, for example, in the N₁ₛ XPS spectrum of free oleylamine which interacts with the nanoparticle through the amino group and has a value of approx. 400 eV in both free and joined cases (Goel, V. Thesis 2011, McGill University, Montreal).

### Example 7: XPS characterization of UCNP@ligand_{2-chloroacetic} nanoparticles.

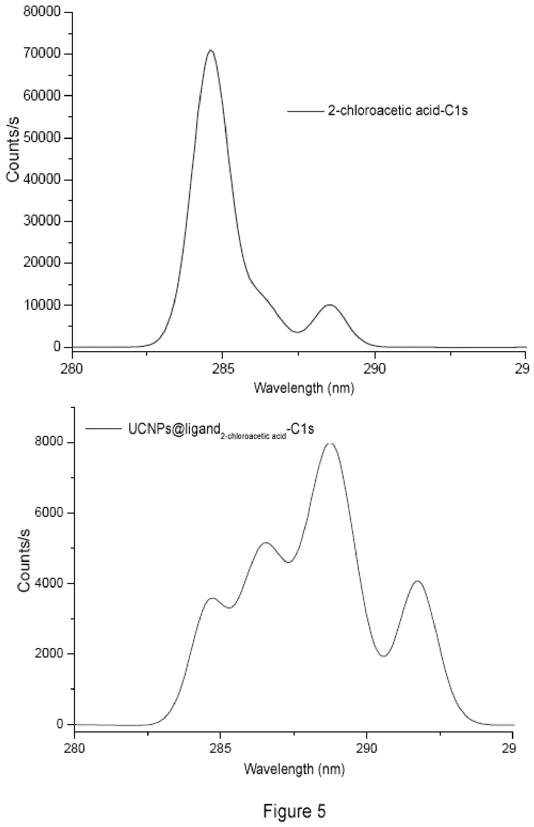

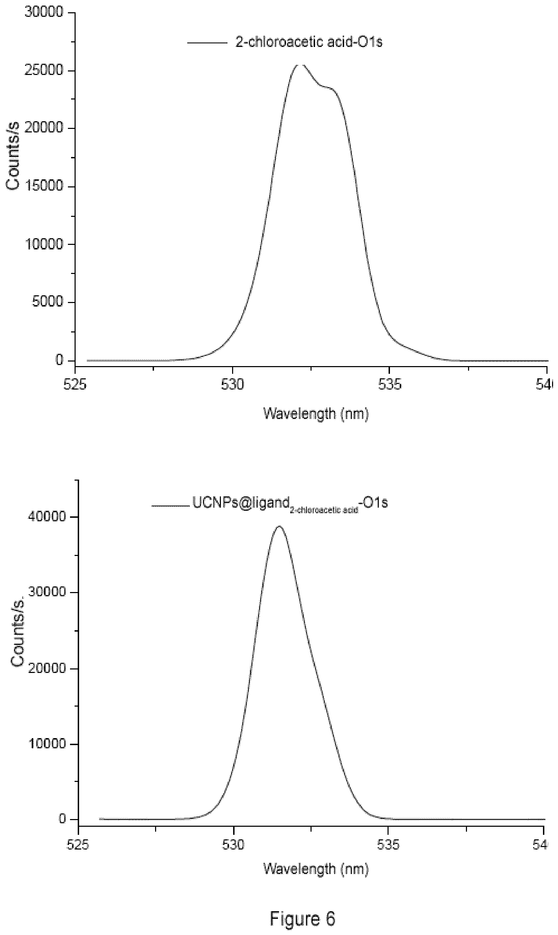

Similarly to Example 6, the C₁ₛ XPS spectra for UCNP@ligand₂-_{chloroacetic} nanoparticles (Figure 5, bottom) prepared as described in Example 4 and for the organic starting compound, 2-chloroacetic acid, (Figure 5, top), were obtained for comparison. The XPS spectra O₁ₛ were also obtained for the nanoparticles UCNP@ligand_{2-chloroacetic} (Figure 6, bottom) prepared as described in the Example 4 and for the organic starting compound, 2-chloroacetic acid, (Figure 6, top).

The significant changes in the C₁ₛ and O₁ₛ energy values between the organic starting compound, 2-chloroacetic acid, and the organic ligand of the UCNP@ligand_{2-chloroacetic} nanoparticle must be pointed out. Like in Example 6, this shift is consistent with a chemical reaction between the organic starting compound, 2-chloroacetic acid, and the surface of the nanoparticle, which allows the ligand to remain on the surface of the nanoparticle in strongly acidic media.

### Example 8: Preparation of a colorless, transparent composite material by sol-gel encapsulation of UCNP@ligand_{2-chloroacetamide} nanoparticles in an acidic medium

A silica sol-gel was prepared for encapsulating UCNP@ligand_{2-chloroacetamide} nanoparticles, such that the pigment remained in dissolution with EtOH at a concentration of 0.6 g/L.

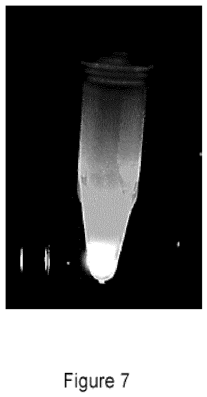

300 mg UCNP@ligand_{2-chloroacetamide} nanoparticles were added to 50 mL of EtOH under stirring and left to homogenize for a few minutes. Next, 24 mL of TEOS were added and 7.2 mL of deionized water were then added dropwise to favor the mixing of TEOS with water and allow hydrolysis reactions to occur. Finally, hydrochloric acid (< 0.1 mL) was also incorporated dropwise until reaching a pH of about 4. Stirring was stopped for at least 1 hour. The resulting solution was only left to dry at room temperature for 48 hours. About 7.5 g of powder were obtained. The UCNP@ligand_{2-chloroacetamide}/sol-gel ratio obtained was 1/25, emitting a green color (Figure 7). The comparison of the emission of 1 mg of UCNP@ligand_{2-chloroacetamide} and the emission of 1 mg of the composite material demonstrated that the composite material is 25 times more emissive. It can therefore be deduced that sol-gel treatment increased the emission capacity of UCNPs by 625-fold.

### Example 9: Preparation of a composite material by means of a sol-gel synthesis process with UCNP@ligand_{2-chloroacetamide} particles in a basic medium

For encapsulating UCNP@ligand_{2-chloroacetamide} nanoparticles, a sol-gel was made in a basic medium using a Zn precursor.

7.5 g of zinc acetate were added to 50 mL of ethanol under stirring, adding 20 mg of nanoparticles, and it was left to homogenize for a few minutes until the acetate was dissolved in the alcohol. The nanoparticle dispersion was then added and stirred for at least 2 minutes. Next, 3 mL of ethanolamine were added dropwise to favor mixing with the ethanol and allow the reactions with zinc acetate to occur. The ethanolamine acted as a solution stabilizer and catalyst. Once the ethanolamine is added, the solution must have a pH of 7 or greater. Stirring was stopped for at least 1 hour. The resulting solution was left to dry at room temperature for 48 hours until most of the ethanol had evaporated. After this time, a highly viscous liquid was obtained and it was treated with heat at 300°C for the ZnO structure to form in 1 hour for greater crystallinity and residue removal. About 2.8 g of powder emitting a pinkish color were obtained after heat treatment.

## Claims

1. Luminescent upconversion nanoparticles of formula MLnF₄:RE⁺³, where M is an alkaline metal, Ln is a lanthanide element or an analog thereof and RE⁺³ is an ion of rare earth elements or combinations thereof, **characterized in that** said nanoparticles comprise organic ligands bound to the surface thereof by means of covalent bonds; wherein said organic ligands are selected from 2-chloroacetamide, 2-bromoacetamide, 2-chloroacetic acid, 2-bromoacetic acid and ethyl 2-chloroacetate.

2. Nanoparticles according to claim 1 with a mean size ≤500 nm.

3. Nanoparticles according to any of claims 1 to 2, wherein the nanoparticles have a core/shell structure.

4. Nanoparticles according to any of claims 1 to 3, where M is Na or Li.

5. Nanoparticles according to any of claims 1 to 4, where RE³⁺ is Yb³⁺, Er³⁺, Tm³⁺, Gd³⁺ or combinations thereof.

6. A method for preparing luminescent upconversion nanoparticles of formula MLnF₄:RE⁺³, where M is an alkaline metal, Ln is a lanthanide element or an analog thereof and RE⁺³ is an ion of rare earth elements or combinations thereof, **characterized in that** said nanoparticles comprise organic ligands bound to the surface thereof by means of covalent bonds, which method comprises the steps of:
a) adding to a polar solvent precursor nanoparticles of formula MLnF₄:RE⁺³, where M is an alkaline metal, Ln is a lanthanide element or an analog thereof and RE⁺³ is an ion of rare earth elements or combinations thereof, **characterized in that** said nanoparticles comprise on their surface -O⁻, -OH, -HOH groups or combinations thereof;
b) adding a base in excess to the mixture resulting from step (a); and
c) after removing the excess base from the mixture resulting from step (b), adding a compound of formula X-CH₂CO-Z; wherein the compound of formula X-CH₂CO-Z is selected from 2-chloroacetamide, 2-bromoacetamide, 2-chloroacetic acid, 2-bromoacetic acid, and ethyl 2-chloroacetate.

7. The method for preparing nanoparticles according to claim 6, wherein the polar solvent of step (a) is dimethylformamide, acetonitrile, tetrahydrofuran or a mixture thereof.

8. The method for preparing nanoparticles according to any of claims 6 to 7, wherein step (a) takes place at a temperature from about 25°C to about 80°C.

9. The method for preparing nanoparticles according to any of claims 6 to 8, wherein the base in excess of step (b) is an inorganic base, preferably NaOH.

10. The method for preparing nanoparticles according to any of claims 6 to 9, wherein the compound of formula X-CH₂CO-Z of step (c) is 2-chloroacetamide or 2-chloroacetic acid.

11. Luminescent upconversion nanoparticles of formula MLnF₄:RE⁺³, where M is an alkaline metal, Ln is a lanthanide element or an analog thereof and RE⁺³ is an ion of rare earth elements or combinations thereof, which comprise organic ligands bound to the surface thereof by means of covalent bonds; wherein the organic ligands are selected from 2-chloroacetamide, 2-bromoacetamide, 2-chloroacetic acid, 2-bromoacetic acid and ethyl 2-chloroacetate and can be obtained by means of the method according to any of claims 6 to 10.

12. Use of the luminescent upconversion nanoparticles according to any of claims 1 to 5 and 11 as fluorescent markers or photosensitizers.

13. Use of the luminescent upconversion nanoparticles according to claim 12 in the biological or medical-pharmaceutical sector.

14. A luminescent composite material comprising the luminescent upconversion nanoparticles according to any of claims 1 to 5 and 11.

15. The luminescent composite material according to claim 14, wherein the composite material is ceramic.

16. The luminescent composite material according to any of claims 14 and 15, wherein the composite material is transparent.

17. The luminescent composite material according to any of claims 14 to 16 which can be obtained by means of a sol-gel synthesis process.

18. Use of the luminescent composite material according to any of claims 14 to 17 as a fluorescent marker or photosensitizer.

19. Use of the luminescent composite material according to any of claims 14 to 17 in the paper, plastic, packaging, textile, automotive, biological, medical-pharmaceutical, document security, tracer, ceramic pigment, optoelectronics or photovoltaic sectors.

## Patentansprüche

1. Lumineszente hochkonvertierte Nanopartikel der Formel MLnF₄:RE⁺³, wobei M ein Alkalimetall ist, Ln ein Lanthanidelement oder ein Analogon davon ist und RE⁺³ ein Ion von Seltenerdelementen oder Kombinationen davon ist, **dadurch gekennzeichnet, dass** die Nanopartikel organische Liganden umfassen, die mittels kovalenter Bindungen an deren Oberfläche gebunden sind; wobei die organischen Liganden aus 2-Chloracetamid, 2-Bromacetamid, 2-Chloressigsäure, 2-Bromessigsäure und Ethyl-2-chloracetat ausgewählt sind.

2. Nanopartikel nach Anspruch 1 mit einer mittleren Größe ≤500 nm.

3. Nanopartikel nach einem der Ansprüche 1 oder 2, wobei die Nanopartikel eine Kern/Schalen-Struktur aufweisen.

4. Nanopartikel nach einem der Ansprüche 1 bis 3, wobei M Na oder Li ist.

5. Nanopartikel nach einem der Ansprüche 1 bis 4, wobei RE³⁺ Yb³⁺, Er³⁺, Tm³⁺, Gd³⁺ oder eine Kombination davon ist.

6. Verfahren zur Herstellung von lumineszenten hochkonvertierten Nanopartikeln der Formel MLnF₄:RE⁺³, wobei M ein Alkalimetall ist, Ln ein Lanthanidelement oder ein Analogon davon ist und RE⁺³ ein Ion von Seltenerdelementen oder eine Kombination davon ist, **dadurch gekennzeichnet, dass** die Nanopartikel organische Liganden umfassen, die mittels kovalenter Bindungen an deren Oberfläche gebunden sind, wobei das Verfahren die folgenden Schritte umfasst:
a) Zugabe von Nanopartikeln der Formel MLnF₄:RE⁺³, wobei M ein Alkalimetall, Ln ein Lanthanidelement oder ein Analogon davon und RE⁺³ ein Ion von Seltenerdelementen oder eine Kombination davon ist, zu einem polaren Lösungsmittelvorläufer, **dadurch gekennzeichnet, dass** die Nanopartikel auf ihrer Oberfläche -O⁻,-OH-, HOH-Gruppen oder Kombinationen davon aufweisen;
b) Hinzufügen einer Base im Überschuss zu der aus Schritt (a) resultierenden Mischung; und
c) nach Entfernung der überschüssigen Base aus dem in Schritt (b) erhaltenen Gemisch, Zugabe einer Verbindung der Formel X-CH₂CO-Z; wobei die Verbindung der Formel X-CH₂CO-Z ausgewählt ist aus 2-Chloracetamid, 2-Bromacetamid, 2-Chloressigsäure, 2-Bromessigsäure und Ethyl-2-chloracetat.

7. Verfahren zur Herstellung von Nanopartikeln nach Anspruch 6, wobei das polare Lösungsmittel von Schritt (a) Dimethylformamid, Acetonitril, Tetrahydrofuran oder eine Mischung davon ist.

8. Verfahren zur Herstellung von Nanopartikeln nach einem der Ansprüche 6 bis 7, wobei Schritt (a) bei einer Temperatur von etwa 25°C bis etwa 80°C stattfindet.

9. Verfahren zur Herstellung von Nanopartikeln nach einem der Ansprüche 6 bis 8, wobei die überschüssige Base aus Schritt (b) eine anorganische Base, vorzugsweise NaOH, ist.

10. Verfahren zur Herstellung von Nanopartikeln nach einem der Ansprüche 6 bis 9, wobei die Verbindung der Formel X-CH₂CO-Z aus Schritt (c) 2-Chloracetamid oder 2-Chloressigsäure ist.

11. Lumineszente hochkonvertierte Nanopartikel der Formel MLnF₄:RE⁺³, worin M ein Alkalimetall ist, Ln ein Lanthanidelement oder ein Analogon davon ist und RE⁺³ ein Ion von Seltenerdelementen oder Kombinationen davon ist, die organische Liganden umfassen, die an deren Oberfläche mittels kovalenter Bindungen gebunden sind; wobei die organischen Liganden aus 2-Chloracetamid, 2-Bromacetamid, 2-Chloressigsäure, 2-Bromessigsäure und Ethyl-2-chloracetat ausgewählt sind und mittels des Verfahrens nach einem der Ansprüche 6 bis 10 erhalten werden können.

12. Verwendung der lumineszenten hochkonvertierten Nanopartikel nach einem der Ansprüche 1 bis 5 und 11 als Fluoreszenzmarker oder Photosensibilisatoren.

13. Verwendung von lumineszenten hochkonvertierten Nanopartikeln nach Anspruch 12 im biologischen oder medizinischpharmazeutischen Bereich.

14. Lumineszenten Verbundmaterial, umfassend die lumineszenten hochkonvertierten Nanopartikel nach einem der Ansprüche 1 bis 5 und 11.

15. Lumineszentes Verbundmaterial nach Anspruch 14, wobei das Verbundmaterial keramisch ist.

16. Das lumineszente Verbundmaterial nach einem der Ansprüche 14 und 15, wobei das Verbundmaterial transparent ist.

17. Das lumineszente Verbundmaterial nach einem der Ansprüche 14 bis 16, das durch ein Sol-Gel-Syntheseverfahren erhalten werden kann.

18. Verwendung des lumineszienten Verbundmaterials nach einem der Ansprüche 14 bis 17 als Fluoreszenzmarker oder Photosensibilisator.

19. Verwendung des lumineszienten Verbundmaterials nach einem der Ansprüche 14 bis 17 in den Bereichen Papier, Kunststoff, Verpackung, Textil, Automobile, Biologie, Medizin-Pharmazie, Dokumentensicherheit, Tracer, keramische Pigmente, Optoelektronik oder Photovoltaik.

## Revendications

1. Nanoparticules luminescentes à conversion ascendante de formule MLnF₄:RE⁺³, dans laquelle M est un métal alcalin, Ln est un élément lanthanide ou un analogue de celui-ci et RE⁺³ est un ion d'éléments de terres rares ou des combinaisons de ceux-ci, **caractérisées en ce que** lesdites nanoparticules comprennent des ligands organiques liés à leurs surfaces au moyen de liaisons covalentes ; lesdits ligands organiques étant choisis parmi le 2-chloroacétamide, le 2-bromoacétamide, l'acide 2-chloroacétique, l'acide 2-bromoacétique et le 2-chloroacétate d'éthyle.

2. Nanoparticules selon la revendication 1, ayant une taille moyenne ≤500 nm.

3. Nanoparticules selon l'une quelconque des revendications 1 à 2, dans lesquelles les nanoparticules ont une structure cœur / coquille.

4. Nanoparticules selon l'une quelconque des revendications 1 à 3, dans lequel M est Na ou Li.

5. Nanoparticules selon l'une quelconque des revendications 1 à 4, dans lequel RE³⁺ est Yb³⁺, Er³⁺, Tm³⁺, Gd³⁺ ou des combinaisons de ceux-ci.

6. Un procédé de préparation de nanoparticules luminescentes à conversion ascendante de formule MLnF₄:RE⁺³, dans laquelle M est un métal alcalin, Ln est un élément lanthanide ou un analogue de celui-ci et RE⁺³ est un ion d'éléments de terres rares ou des combinaisons de ceux-ci, **caractérisé en ce que** lesdites nanoparticules comprennent des ligands organiques liés à leurs surfaces au moyen de liaisons covalentes, lequel procédé comprend les étapes:
a) ajouter à un précurseur de solvant polaire des nanoparticules de formule MLnF₄:RE⁺³, dans laquelle M est un métal alcalin, Ln est un élément lanthanide ou un analogue de celui-ci et RE⁺³ est un ion d'éléments de terres rares ou des combinaisons de ceux-ci, **caractérisé en ce que** lesdites nanoparticules comprennent à leur surface des groupes -O⁻ , -OH, -HOH ou des combinaisons de ceux-ci ;
b) ajouter une base en excès au mélange résultant de l'étape (a) ; et
c) après avoir retiré l'excès de base du mélange résultant de l'étape (b), ajouter un composé de formule X-CH₂CO-Z ; le composé de formule X-CH₂CO-Z est choisi parmi le 2-chloroacétamide, le 2-bromoacétamide, l'acide 2-chloroacétique, l'acide 2-bromoacétique et le 2-chloroacétate d'éthyle.

7. Le procédé de préparation de nanoparticules selon la revendication 6, dans lequel le solvant polaire de l'étape (a) est le diméthylformamide, l'acétonitrile, le tétrahydrofurane ou un mélange de ceux-ci.

8. Le procédé de préparation de nanoparticules selon l'une quelconque des revendications 6 à 7, dans lequel l'étape (a) est mise en œuvre à une température d'environ 25° C à environ 80° C.

9. Le procédé de préparation de nanoparticules selon l'une quelconque des revendications 6 à 8, dans lequel la base en excès de l'étape (b) est une base inorganique, de préférence NaOH.

10. Le procédé de préparation de nanoparticules selon l'une quelconque des revendications 6 à 9, dans lequel le composé de formule X-CH₂CO-Z de l'étape (c) est le 2-chloroacétamide ou l'acide 2-chloroacétique.

11. Nanoparticules luminescentes à conversion ascendante de formule MLnF₄:RE⁺³, dans laquelle M est un métal alcalin, Ln est un élément lanthanide ou un analogue de celui-ci et RE⁺³ est un ion d'éléments de terres rares ou des combinaisons de ceux-ci, qui comprennent des ligands organiques liés à leurs surfaces au moyen de liaisons covalentes ; les ligands organiques étant choisis parmi le 2-chloroacétamide, le 2-bromoacétamide, l'acide 2-chloroacétique, l'acide 2-bromoacétique et le 2-chloroacétate d'éthyle et peuvent être obtenus au moyen du procédé selon l'une quelconque des revendications 6 à 10.

12. Utilisation des nanoparticules à conversion ascendante luminescentes selon l'une quelconque des revendications 1 à 5 et 11 en tant que marqueurs fluorescents ou photosensibilisateurs.

13. Utilisation des nanoparticules à conversion ascendante luminescentes selon la revendication 12 dans le secteur biologique ou médico-pharmaceutique.

14. Un matériau composite luminescent comprenant les nanoparticules à conversion ascendante luminescentes selon l'une quelconque des revendications 1 à 5 et 11.

15. Le matériau composite luminescent selon la revendication 14, dans lequel le matériau composite est de la céramique.

16. Le matériau composite luminescent selon l'une quelconque des revendications 14 et 15, dans lequel le matériau composite est transparent.

17. Le matériau composite luminescent selon l'une quelconque des revendications 14 à 16 qui peut être obtenu au moyen d'un procédé de synthèse sol-gel.

18. Utilisation du matériau composite luminescent selon l'une quelconque des revendications 14 à 17 en tant que marqueur fluorescent ou photosensibilisateur.

19. Utilisation du matériau composite luminescent selon l'une quelconque des revendications 14 à 17 dans les secteurs du papier, du plastique, de l'emballage, du textile, de l'automobile, biologique, médico-pharmaceutique, de la sécurité de document, des traceurs, des pigments céramiques, de l'optoélectronique ou du photovoltaique.
